# FASCICULE DE BREVET EUROPEEN

(11) **EP 1 307 593 B1**
(45) Date de publication et mention de la délivrance du brevet: **15.08.2007**
(21) Numéro de dépôt: 01960869.4
(22) Date de dépôt: 06.08.2001
(51) Int. Cl.: C12Q 1/68

(54) **DEPISTAGE D'UN NOUVEAU SYNDROME HEPATIQUE ET SES APPLICATIONS**
SCREENING EINER NEUEN LEBER-SYNDROMS UND SEINE ANWENDUNGEN
SCREENING OF A NOVEL HEPATIC SYNDROME AND ITS USES

(30) Priorité: 08.08.2000 FR 0010428
(43) Date de publication de la demande: 07.05.2003
(73) Titulaire: ASSISTANCE PUBLIQUE, HOPITAUX DE PARIS, 75004 Paris (FR)
(72) Inventeur: POUPON, Raoul, F-75013 Paris (FR); HERMELIN, Brigitte, F-78000 Versailles (FR); ROSMORDUC, Olivier, F-75011 Paris (FR)
(74) Mandataire: Vialle-Presles, Marie José
(86) Numéro de dépôt international: PCT/FR2001/002553
(87) Numéro de publication internationale: WO 2002/012556

(56) Documents cités:
- EP-A- 0 086 705
- DIXON PH ET AL: "Heterogeneous MDR3 missense mutation associated with intrahepatic cholestasis of pregnancy: evidence for a defect in protein trafficking" HUMAN MOLECULAR BIOLOGY, vol. 9, no. 8, mai 2000 (2000-05), pages 1209-17, XP002166232
- DE VREE JML. ET AL: "Mutations in the MDR3 gene cause progressive familial intrahepatic cholestasis" PROC. NATL. ACAD. SCI. USA, vol. 95, 6 janvier 1998 (1998-01-06), pages 282-287, XP002142063
- JACQUEMIN E ET AL: "Heterozygous non-sense mutation of the MDR3 gene in familial intrahepatic cholestasis of pregnancy" THE LANCET, vol. 353, 16 janvier 1999 (1999-01-16), pages 210-11, XP002166234
- JACQUEMIN E ET AL: "Genetic basis of progressive familial intrahepatic cholestasis" JOURNAL OF HEPTOLOGY, vol. 31, no. 2, août 1999 (1999-08), pages 377-81, XP000993154
- JACQUEMIN E ET AL: "MDR3 deficiency in patients with progressive familial intrahepatic cholestasis with high serum gamma-glutamyl transferase 8GGT) activity (PFIC3)" JOURNAL OF PEDIATRIC GASTROENTEROLOGY AND NUTRITION, vol. 31, 5 - 9 août 2000, page s207 XP000993184
- ROSMORDUC O ET AL: "MDR3 gene defect in adults with symtomatic intrahepatic and gallbladder cholesterol cholelithiasis" GASTROENTEROLOGY, vol. 120, 18 avril 2001 (2001-04-18), pages 1459-1467, XP002166235
- TOMIDA S. ET AL: 'Long-term urodeoxycholic acid therapy is associated with reduced risk of biliary pain and acute cholecystitis in patients with gallbladder stones; a cohort analysis' HEPATOLOGY vol. 30, no. 1, Juillet 1999, pages 6 - 13
- TINT G.S. ET AL: 'Lithotripsy plus ursodiol is superior to ursodiol alone for cholesterol gallstones' GASTROENTEROLOGY vol. 102, no. 6, Juin 1992, pages 2042 - 2049
- LINCKE ET AL: 'Structure of the human MDR3 gene and physical mapping of the human MDR locus.' J BIOL CHEM vol. 266, no. 8, 15 Mars 1991, pages 5303 - 5310
- DATABASE ENTREZ NUCLEOTIDE [en ligne] NCBI VAN DER BLIEK ET AL Database accession no. M23234
- DATABASE [en ligne] 'Listage de séquences, pages 1-21 ("Annexe 2")'
- DATABASE [en ligne] 'Listage de séquences pages 1-5 ("Annexe 3")'
- VAN DER BLIEK ET AL: 'Sequence of mdr3 cDNA encoding a human P-glycoprotein' GENE vol. 71, 1988, pages 401 - 411

## Description

La présente invention est relative au dépistage d'un syndrome hépatique survenant chez l'adulte jeune et associant une microlithiase biliaire cholestérolique, une cholestase intra-hépatique et une ou plusieurs mutations du gène *MDR3.*

La présente invention est également relative au traitement dudit syndrome.

La lithiase biliaire cholestérolique est caractérisée par la présence de calculs de cholestérol ou de bilirubinate de calcium. La lithiase cholestérolique est la forme la plus fréquente de lithiase biliaire (80 %). Elle se caractérise par la formation de calculs de cholestérol dans la vésicule biliaire, due à un excès de cholestérol biliaire par rapport aux molécules solubilisantes que sont les acides biliaires et les phospholipides. Sa prévalence est estimée, tout âge confondu à 10 à 20 % de la population dans les pays industrialisés. La prévalence est plus grande chez la femme et augmente considérablement avec l'âge (40 % chez la femme après 60 ans). La lithiase vésiculaire peut être source de complications cholécystite, cancer de la vésicule, migration de calculs dans le cholédoque, source d'angiocholite et de pancréatite. Le traitement de cette maladie repose principalement sur la chirurgie : cholécystectomie par laparotomie ou coelioscopie, drainage biliaire avec extraction peropératoire ou perendoscopique des calculs cholédociens. Le traitement médical qui consiste en l'administration d'acide chénodésoxycholique et/ou d'acide ursodésoxycholique, est réservé aux formes de lithiases biliaires cholestéroliques vésiculaires non compliquées.

Les cholestases biliaires chroniques sont caractérisées par un défaut de passage de la bile du foie vers les voies biliaires extra-hépatiques et l'intestin. Les causes et la physiopathologie sont variées (Poupon et al., 2000, J. Hepatol., 32, 129-140). La plupart des cholestases biliaires chroniques peuvent évoluer vers une cirrhose biliaire et une insuffisance hépatocellulaire nécessitant une transplantation hépatique.

Chez l'enfant par exemple, on observe une cholestase dans l'atrésie biliaire, dans le syndrome d'Alagille ou dans les cholestases intra-hépàtiques familiales progressives (PFIC). De manière générale, on observe des anomalies génétiques dans ces différentes maladies.

Les cholestases intra-hépatiques familiales (PFIC) sont des maladies récessives infantiles qui se caractérisent par des jaunisses intermittentes, une cholestase sévère et une cirrhose, dont l'issue est fatale au cours des dix premières années de la vie.

Ces maladies se manifestent sous plusieurs formes cliniques, dont deux sont déterminées par des mutations présentes sur des gènes codant pour des transporteurs ABC. La maladie de Byler ou cholestase intra-hépatique familiale progressive de type 1, qui se caractérise par une cholestérolémie et une activité γ-glutamyl transférase sérique normales, a été la première décrite. Elle est causée par la mutation du gène *FIC1,* une ATPase de type V, impliquée dans le transport des phospholipides, de la paroi externe vers la paroi interne de différentes membranes cellulaires. De manière plus précise, la PFIC de type 1 est caractérisée par des épisodes récurrents de jaunisse, un prurit sévère, une activité γ-GT et des taux de cholestérol normaux, des concentrations élevées en acides biliaires sériques et des taux faibles en acides biliaires dans la bile. Le locus de la PFIC1 a été trouvé dans le chromosome 18q21-q22 ; cinq mutations ont été identifiées : une délétion, un exon supprimé et trois mutations faux-sens ; ces mutations faux-sens concernent des domaines hautement conservés dans les ATPases de type V (E. Jacquemin et al., J. Hepatol., 1999, 31, 377-381).

La cholestase familiale de type 2 présente les mêmes manifestations cliniques que la maladie de Byler mais elle est causée par des mutations dans le gène *FIC2,* qui code pour le transporteur BSEP (*Bile Salt Export Pump*). Ce gène est l'homologue du gène murin *SPGP* dont le produit, exprimé exclusivement dans les hépatocytes, est impliqué dans le transport des sels biliaires. La PFIC de type 2 est caractérisée par un prurit sévère, une activité γ-GT et un taux de cholestérol sérique normaux, des concentrations élevées en acides biliaires sériques et des taux très bas d'acides biliaires dans la bile. Le locus de la PIFC2 a été trouvé dans le chromosome 2q24: 10 mutations été identifiées, incluant des délétions et des mutations faux-sens impliquant des domaines importants du transporteur BSEP (E. Jacquemin et al., J. Hepatol., 1999, 31, 377-381).

Les malades atteints de cholestase intra-hépatique familiale progressive de type 3 (PFIC3) présentent des mutations dans le gène *MDR3.* La PFIC de type 3 se distingue des autres PFIC en particulier par une activité γ-glutamyl transférase sérique élevée. La famille MDR (*Multi Drug Resistance*) de transporteurs ABC est une famille multigénique de protéines homologues, dont certaines (MDR3) n'interviennent pas dans la résistance aux drogues et sont des transporteurs de phosphatidylcholine. Le gène *MDR3,* mis en évidence en 1993, est un translocateur de phospholipides. Exprimé dans l'hépatocyte, il assure la translocation de phosphatidylcholine, entraînant ainsi la sécrétion de ce phospholipide dans la bile.

Les souris chez lesquelles le gène équivalent à *MDR3* a été invalidé *mdr2* (-/-) présentent un défaut de sécrétion de phospholipides dans la bile, qui provoque des altérations physiologiques proches de celles de patients atteints de cholestase progressive intra-hépatique familiale de type 3. Deux patients atteints de cette maladie et portant des mutations sur les deux allèles de *MDR3* ont été récemment décrits ; le profil histopathologique des deux patients est similaire à celui observé chez les souris *mdr2* (-/-) (J. Marleen L. de Vree et al., PNAS, 1998, 95, 1, 282-287). De manière plus précise, au niveau histologique, une fibrose portale, une prolifération des canaux biliaires et la présence d'un infiltrat inflammatoire sont observés. En revanche, il n'est pas observé de microlithiase biliaire cholestérolique chez ces patients atteints de PFIC3 ou chez les souris *mdr2* (-/-). Au niveau génique, chez le premier patient, une délétion homozygote de 7 pb a été observée, commençant à l'acide aminé 132, entraînant un décalage du cadre de lecture et introduisant un codon stop, 29 codons en aval ; le second patient est homozygote pour une mutation non-sens dans le codon 957 (C/T) qui introduit un codon stop (TGA) : cette mutation supprime un site de restriction *TaqI.* Dans une telle pathologie, des protéines tronquées, non actives, dans lesquelles au moins un motif ABC manque, sont exprimées. En outre, des mutations non-sens additionnelles et des mutations faux-sens, associées avec des taux très bas de phospholipides biliaires ont aussi été observées.

Des cholestases n'apparaissant que lors de la grossesse ont également été observées chez des patientes présentant une mutation hétérozygote du gène *MDR3* ; dans de tels cas, on n'a pas observé de microlithiase (E. Jacquemin et al., The Lancet, 1999,353,210-211; Dixon et al., Human Molecular Genetics, 2000, 9, 1209-1217).

De manière plus précise, chez ces patientes, on observe :
- soit une délétion hétérozygote d'un nucléotide (1712deIT) commençant à l'acide aminé 571, entraînant un décalage du cadre de lecture introduisant un codon stop, 15 codons en aval : cette mutation entraînant l'expression d'une protéine tronquée non active a été détectée chez des femmes enceintes issues d'une famille atteinte de PFIC3 (Jacquemin et al., The Lancet, 1999, précité)
- soit une mutation, faux-sens, au niveau de l'exon 14 (A546D) : cette mutation a été détectée chez des femmes enceintes sans antécédent familial de PFIC (Dixon et al., précité).

De manière surprenante, les Inventeurs ont trouvé qu'il existe chez des adultes jeunes (20-40 ans), des pathologies qui se distinguent de la PFIC de type 3, qui sont déclenchées dans des situations telles que la grossesse, la prise d'hormones sexuelles, le jeûne prolongé, la nutrition parentérale prolongée, des interventions chirurgicales ou par des co-facteurs hépatotoxiques (obésité, diabète, prise de médicaments susceptibles de modifier la composition de la bile : fibrates ou diurétiques, en particulier) et dans lesquelles on observe en association :
- une microlithiase biliaire cholestérolique
- une cholestase intra-hépatique et
- au moins une mutation du gène *MDR3.*

Dans ce syndrome, on observe un défaut de phospholipides biliaires (anomalie de la sécrétion des phospholipides biliaires), limitant la solubilisation du cholestérol et entraînant la précipitation du cholestérol sous forme de microcalculs *in situ* au sein des petites voies biliaires et dans les formes extrêmes au sein de l'ensemble de l'arbre biliaire.

Ces microcalculs qui peuvent être détectés par l'examen ultrasonographique du foie présentent les caractéristiques suivantes :
- ils sont observés sous forme de multiples calculs de petite taille (< 2 mm) présents dans les canaux biliaires intra-hépatiques,
- ils sont sensibles au traitement par l'acide ursodésoxycholique (AUDC) ; ce traitement permet la disparition rapide des symptômes, contrairement à la cholécystectomie qui entraîne une récidive de la maladie. Cette récidive peut toutefois être prévenue par l'administration d'AUDC, et
- ils se distinguent des calculs vésiculaires par leur petite taille, leur localisation et leur sensibilité au traitement par l'AUDC.

La très forte diminution de la concentration en phospholipides biliaires et la présence de cristaux de cholestérol sensibles au traitement par l'AUDC, associées à une mutation du gène *MDR3,* caractérisent ce syndrome.

Le dépistage et la prévention de cette pathologie, distincte des PFIC de type 3, chez des sujets ayant des antécédents personnels ou familiaux de lithiase vésiculaire ou d'atteintes hépatiques inexpliquées (anomalies biologiques portant sur les tests de cholestase : augmentation de l'activité sérique des γ-glutamyltranspeptidases, transaminases) sont cruciaux. En effet, 10 à 20 % des cholestases gravidiques pourraient en fait correspondre à ce syndrome et sa morbidité, dont les avortements à répétition, pourrait ainsi être évitée, dans le cas d'un bon dépistage et d'une prévention efficace.

C'est pourquoi les Inventeurs se sont donnés pour but de pourvoir à un test de dépistage de cette pathologie, qui se distingue de la cholestase intra-hépatique familiale progressive de type 3 (PFIC3) tant cliniquement que biochimiquement, de manière à prévenir ses effets néfastes notamment chez la femme en état de procréer.

La présente invention a pour objet un procédé de dépistage d'un syndrome hépatique associant (i) une cholestase intra-hépatique, (ii) une microlithiase biliaire cholestlérolique et (iii) au moins une mutation du gène MDR3, caractérisé en ce qu'il comprend au moins la détection, à partir d'un échantillon d'acide nucléique extrait de cellules mononucléées du sang périphérique, d'au moins une mutation hétérozygote du gène *MDR3* et/ou d'une mutation homozygote n'abolissant pas l'expression de la protéine exprimée par ledit gène à activité de transporteur de la phosphatidylcholine, chez des sujets adultes associant une microlithiase biliaire cholestérolique et une cholestase intra-hépatique.

Au sens de la présente invention, on entend par mutation n'abolissant pas l'expression de la protéine ayant une activité de transporteur de la phosphatidylcholine, aussi bien les mutations qui entraînent l'expression d'une protéine ayant une activité résiduelle de transporteur de la phosphatidylcholine que des mutations qui diminuent le taux d'expression de la protéine normale (mutation dans le promoteur par exemple). Ces différentes mutations n'induisent aucun symptôme hépato-biliaire chez l'enfant, mais l'apparition, chez l'adulte jeune, de symptômes associant une microlithiase biliaire cholestérolique et une cholestase intra-hépatique, induits par des facteurs additionnels liés à l'hôte et /ou à l'environnement.

Conformément à l'invention, la dite mutation est de préférence située dans l'exon 6 et/ou dans l'exon 9 et/ou dans l'exon 12.

Selon un mode de mise en oeuvre avantageux dudit procédé, il comprend la détection d'une mutation sélectionnée dans le groupe constitué par une mutation dans l'exon 6 au niveau du nucléotide 523, une mutation dans l'exon 9 au niveau du nucléotide 959 et une mutation dans l'exon 12 au niveau du nucléotide 1327.

De manière plus précise, ledit procédé comprend la détection d'une mutation sélectionnée dans le groupe constitué par :
- une mutation hétérozygote faux-sens dans l'exon 6 au niveau du nucléotide 523 : A/G (ACG/GCG), conduisant à la mutation en acide aminé T175A ; cet acide aminé est situé dans une séquence d'acides aminés conservés, requise pour l'activité ATPase de la protéine MDR3,
- une mutation homozygote faux-sens dans l'exon 9 : au niveau du nucléotide 959 : C/T (TCC/TTC), conduisant à la mutation en acide aminé S320F, qui se situe à l'extrémité du domaine transmembranaire 5 (TM5) et
- une mutation hétérozygote dans l'exon 12 : insertion d'une adénine au niveau du nucléotide 1327 (1327insA) dans le premier domaine de liaison au nucléotide (NBD1) ; cette mutation entraîne un décalage dans le cadre de lecture au niveau du codon 443 et l'apparition d'un codon stop (nt 1339-1341), conduisant à l'expression d'une protéine tronquée de 446 acides aminés.

Selon un autre mode de mise en oeuvre avantageux du procédé selon l'invention, il comprend :
- une première étape d'amplification d'un fragment d'acide nucléique, dans lequel l'une au moins desdites mutations est susceptible d'être observée, à partir des acides nucléiques extraits de cellules mononucléées du sang périphérique, à l'aide d'au moins une amorce sélectionnée dans le groupe constitué par les amorces représentées dans la liste de séquences en annexe sous les numéros SEQ ID NO:1 à SEQ ID NO:13, et
- une deuxième étape de détection de la présence d'au moins l'une desdites mutations, à partir du ou desdits fragments d'amplification obtenus.

Selon une disposition avantageuse de ce mode de mise en oeuvre du procédé, la première étape d'amplification consiste en une amplification PCR directe d'au moins l'un des exons 6, 9 et 12 de l'ADN génomique avec les amorces suivantes, localisées dans les régions introniques flanquantes :
- amorces SEQ ID NO:1 et SEQ ID NO:2, qui permettent d'amplifier un fragment de 321 pb couvrant l'exon 6,
- amorces SEQ ID NO:3 et SEQ ID NO:4, qui permettent d'amplifier un fragment de 210 pb couvrant l'exon 9, et
- amorces SEQ ID NO:5 et SEQ ID NO:6, qui permettent d'amplifier un fragment de 154 pb couvrant l'exon 12.

Selon une autre disposition avantageuse de ce mode de mise en oeuvre du procédé, la première étape d'amplification.consiste en une amplification RT-PCR des ARNm par deux PCR imbriquées, à l'aide de deux paires d'amorces, sélectionnées dans le groupe constitué par les paires suivantes :
- Première paire : SEQ ID NO:7 et SEQ ID NO:8
- Deuxième paire :
   - SEQ ID NO:9 et SEQ ID NO:10
   - SEQ ID NO:11 et SEQ ID NO:10
   - SEQ ID NO:12 et SEQ ID NO: 10 et
   - SEQ ID NO: 13 et SEQ ID NO:10.

De manière plus précise, pour la détection à partir de l'ARNm, le procédé selon l'invention comprend avantageusement :
- une première amplification PCR d'un fragment de 2100 pb (fragment 1) correspondant aux exons 1 à 16 du gène *MDR3* humain avec la paire d'amorces :
   5'-CCTGCCAGACACGCGCGAGGTTC-3' (SEQ ID NO:7) et 5'-CTTCAAGTCCATCGGTTTCCACATC-3' (SEQ ID NO :8), et
   - une deuxième amplification du fragment 1 en utilisant différents couples d'amorces, aptes respectivement à amplifier, un fragment comprenant l'exon 6, un fragment comprenant l'exon 9 et un fragment comprenant l'exon 12.

De manière encore plus précise :
- un fragment de 1746 pb (fragment 2) comprenant l'exon 6 est amplifié avec le couple d'amorces suivant :
   5'-CCTTGTCGCTGCTAAATCC-3' (SEQ ID NO:9) et
   5'-GGCTCTTCTGACACATTTGTG-3' (SEQ ID NO:10)
- un fragment de 1483 pb (fragment 3) comprenant l'exon 9 est amplifié avec le couple d'amorces suivant :
   5-'GGAATTGGTGACAAGGTTGG-3' (SEQ ID NO:11 et SEQ ID NO:10.
- un fragment de 1162 pb (fragment 4) comprenant l'exon 9 est amplifié avec le couple d'amorces suivant :
   5'-GCTATTTCAGCAAACATTTCCATGG -3' (SEQ ID NO:12) et SEQ ID NO:10
- un fragment de 823 pb (fragment 5) comprenant l'exon 12 est amplifié avec le couple d'amorces suivant :
   5'-GCTAACGTCAAGATCTTGAAGG-3' (SEQ ID NO:13) et SEQ ID NO: 10.

Selon encore une autre disposition avantageuse de ce mode de mise en oeuvre du procédé, la deuxième étape de détection de la présence d'au moins l'une desdites mutations à partir du fragment amplifié, est réalisée selon le cas, par l'une des méthodes suivantes :
- séquençage
- restriction enzymatique ou
- techniques de type PCR/PCR/LDR.

Dans le cas où la mutation se traduit par l'apparition ou la disparition d'un site de restriction dans le fragment amplifié, les mutations sont détectées par digestion avec l'enzyme reconnaissant ledit site de restriction, par comparaison avec un témoin ne présentant pas ladite mutation.

Par exemple :
- digestion du fragment d'amplification de 210 pb, tel que défini ci-dessus avec l'enzyme *HinfI* : la détection d'un fragment de 210 pb correspond à la présence de l'allèle sauvage, alors que la détection de deux fragments (142 pb et 48 pb) correspond à la présence de l'allèle muté.
- digestion du fragment d'amplification de 210 pb tel que défini ci-dessus avec l'enzyme *BamHI* : la détection d'un fragment de 210 pb correspond à la présence de l'allèle muté et la détection de deux fragments correspond à la présence de l'allèle sauvage.

Dans le cas où la mutation ne peut pas être détectée par digestion enzymatique, le fragment amplifié par PCR est séquencé selon les techniques classiques ; à titre d'exemple on peut citer la technique des didésoxynucléotides.

Dans le cas où la mutation à détecter correspond à l'insertion ou à la délétion d'une petite séquence, ladite mutation peut être détectée par des techniques de type PCR/PCR/LDR qui reposent sur la détection de la ligation d'amorces spécifiques d'allèle à l'aide d'une ligase thermostable (Favis et al., Nature Biotech., 2000, 18, 561-564).

Un tel procédé peut avantageusement être mis en oeuvre pour l'évaluation du risque d'apparition d'un syndrome associant une cholestase intra-hépatique et une microlithiase biliaire cholestérolique dans les familles à risque de lithiase cholestérolique et/ou ayant des anomalies biologiques hépatiques inexpliquées.

Lorsqu'une mutation, telle que définie ci-dessus, est détectée chez des sujets à risque, ledit procédé d'évaluation peut avantageusement comprendre en outre, la recherche de microcristaux intra-hépatiques de cholestérol, la mesure de l'index de saturation de la bile en cholestérol, la détermination du rapport cholestérol/phospholipides biliaires, le dosage des phospholipides biliaires et l'épreuve de charge à l'acide ursodésoxycholique.

On peut considérer le diagnostic positif du syndrome précité lorsque l'on dépiste l'une des mutations précitées, associée à la présence de microlithiase biliaire, un taux très faible de phospholipides biliaires et à un indice de saturation de la bile en cholestérol supérieur à 1.

La présente invention a également pour objet l'utilisation des différentes amorces telles que définies ci-dessus pour le dépistage d'un syndrome associant une microlithiase biliaire cholestérolique, une cholestase intra-hépatique et au moins une mutation du gène *MDR3.*

Est également décrit un kit pour le dépistage d'un syndrome associant une microlithiase biliaire cholestérolique, une cholestase intra-hépatique et au moins une mutation du gène *MDR3,* caractérisé en ce qu'il comprend du moins deux amorces telles que définies ci-dessus, aptes à la détection d'une mutation du gène *MDR3* humain, telle que définie ci-dessus.

Est également décrit un kit pour l'évaluation du risque d'apparition d'un syndrome associant une microlithiase biliaire cholestérolique, une cholestase intra-hépatique et au moins une mutation du gène *MDR3*, dans une population à risque, caractérisé en ce qu'il comprend outre les amorces aptes à la détection d'une mutation du gène *MDR3* telles que définies ci-dessus, des réactifs pour doser le cholestérol et les phospholipides biliaires.

De manière préférée, lesdits réactifs sont de réactifs enzymatiques.

La présente invention a en outre pour objet l'utilisation de l'acide ursodésoxycholique pour la préparation d'un médicament destiné à traiter un syndrome hépatique associant une microlithiase biliaire cholestérolique, une cholestase intra-hépatique et au moins une mutation du gène *MDR3.*

Outre les dispositions qui précèdent, l'invention comprend encore d'autres dispositions, qui ressortiront de ce qui va suivre, qui se réfère à des exemples de mise en oeuvre de la présente invention ainsi qu'aux dessins annexés dans lesquels :

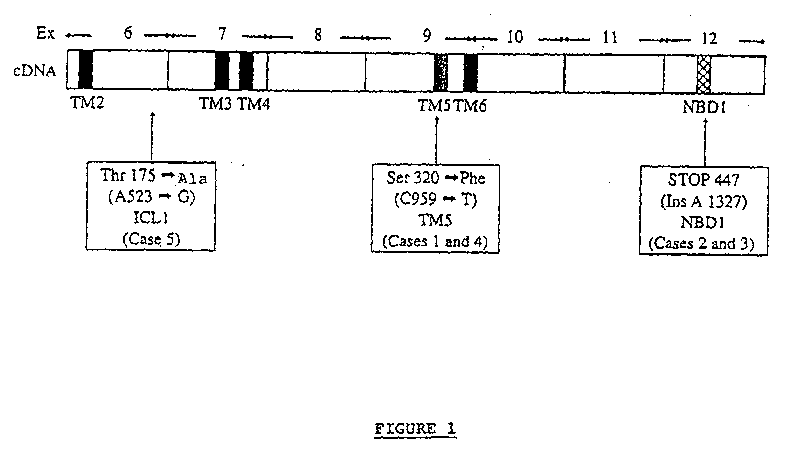

La figure 1 illustre la localisation des différentes mutations sur l'ADNc codant la protéine MDR3. Les exons (Ex) 6 à 12 sont représentés par des boîtes blanches, les boîtes noires correspondent aux domaines transmembranaires (TM) 2 à 6 et la boîte hachurée représente le domaine de fixation des nucléotides (NBD1). La localisation des différentes mutations est représentée par des flèches. ICL1 correspond à la boucle intracellulaire de la protéine MDR3 indispensable à l'activité ATPase de cette protéine.

### Exemple 1 : Matériels et Méthodes

### 1- Caractéristiques des patients inclus dans l'étude

Six patients sans antécédent familial de PFIC ont été étudiés : cinq femmes (patients 1, 2, 4, 5 et 6) et un homme (patient 3). Ils présentent : une cholestase intra-hépatique chronique, une lithiase biliaire de cholestérol, une microlithiase intra-hépatique attestée par échographie et une anomalie du gène *MDR3*; un traitement à l'acide ursodésoxycholique (AUDC) entraîne une disparition complète des symptômes et une normalisation des constantes enzymatiques du foie. Chez les cinq femmes, ce syndrome est apparu lors de la grossesse ou après un traitement par contraceptifs oraux.

L'analyse de la bile montre : une sursaturation en cholestérol, un taux de phospholipides bas et la présence de cristaux de cholestérol.

### 2 - Préparation de l'ADN et de l'ARN.

Les cellules mononucléées du sang circulant sont isolées par centrifugation (Histopaque-1119, Sigma Diagnostics). L'ADN est extrait à partir de 5.10⁶ lymphocytes à l'aide du kit QIAamp (Qiagen-GmbH, Allemagne). L'ARN total est extrait à partir de 5.10⁶ cellules mononucléées du sang périphérique (kit Eurobio, les Ulis, France) conformément aux instructions du fabricant. Les concentrations en ARN et en ADN sont quantifiées par spectrophotométrie.

### 3- Amplification des transcrits du gène MDR par RT-PCR

Une transcription inverse est réalisée dans un volume réactionnel de 20 µl contenant 500 mM de dNTP, 10 mM de DTT, 0.5 U.ml⁻¹ de RNasine (Madison, Wi), 5 mM d'hexamères aléatoires et 10 µg.ml⁻¹ de transcriptase inverse (Gibco BRL, Bethesda, MD). Les extraits d'ARN sont chauffés pendant 5 minutes à 70°C et refroidis sur de la glace avant d'être ajoutés (1 µg) au mélange réactionnel et incubés une heure à 37°C.

L'ADNc obtenu (5 µl, 0,25 µg) est amplifié par la méthode PCR à l'aide d'amorces oligonucléotidiques synthétiques (Genosys, Cambridge, UK). Une première PCR, produisant le fragment 1 de 2100 pb est réalisée dans un milieu de réaction de 25 µl contenant 10 mM de Tris-HCl pH 8.3, 1,5 mM de chlorure de magnésium, 0,001 % de gélatine, 0,05 U de Taq DNA polymérase, 350 µM de chaque dNTP et 0,5 µM de chaque amorce (sens et anti-sens) telle que définie dans le Tableau I ci-après :

**Tableau I: Séquence des amorces et taille des fragments obtenus par amplification RT-PCR du gène MDR3**

| **Fragment** | **Paires d'amorces PCR (5'→3')** | **Position)** | **Position (Exon)** | **Taille du produit (pb)** |
|---|---|---|---|---|
| 1 | CCTGCCAGACACGCGCGAGGTTC (SEQ ID NO:7) | -32-2068 | (1-16) | 2100 |
| | CTTCAAGTCCATCGGTTTCCACATC (SEQ ID NO:8) | | | |
| 2 | CCTTGTCGCTGCTAAATCC (SEQ ID NO:9) | 296-2041 | (5-16) | 1746 |
| | GGCTCTTCTGACACATTTGTG (SEQ ID NO:10) | | | |
| 3 | GGAATTGGTGACAAGGTTGG (SEQ ID NO: 11) | 559-2041 | (9-16) | 1483 |
| | GGCTCTTCTGACACATTTGTG (SEQ ID NO:10) | | | |
| 4 | GCTATTTCAGCAAACATTTCCATGG (SEQ ID NO:12) | 880-2041 | (9-16) | 1162 |
| | GGCTCTTCTGACACATTTGTG (SEQ ID NO:10) | | | |
| 5 | GCTAACGTCAAGATCTTGAAGG (SEQ ID NO:13) | 1219-2041 | (11-16) | 823 |
| | GGCTCTTCTGACACATTTGTG (SEQ ID NO:10) | | | |
| 6 | GAGGCCAACGCCTATGAG (SEQ ID NO:14) | 1522-2041 | (13-16) | 520 |
| | GGCTCTTCTGACACATTTGTG (SEQ ID NO:10) | | | |

La paire d'amorces pour l'amplification du fragment 1 a déjà été décrite (De Vree JM. et al., précité).

L'amplification PCR primaire est réalisée dans les conditions suivantes : une étape de dénaturation initiale à 94° C pendant cinq minutes est suivie de 35 cycles alternant une étape de dénaturation à 94° C pendant trente secondes, une étape d'hybridation des amorces à 68° C pendant trente secondes et une étape d'extension à 72° C pendant deux minutes.

Des PCR imbriquées produisant cinq fragments chevauchants (fragments 2, 3, 4, 5 et 6) sont réalisées en ajoutant 2 µl du produit d'amplification de la PCR primaire à un mélange réactionnel de PCR secondaire (50 µl). Le milieu de réaction est identique à celui décrit ci-dessus (pour les amorces, voir Tableau I).

L'amplification PCR secondaire est réalisée dans les conditions suivantes : une étape initiale de dénaturation à 94° C pendant cinq minutes est suivie de 35 cycles alternant une étape de dénaturation à 94° C pendant trente secondes, une étape d'hybridation des amorces à 60° C pendant trente secondes et une étape d'extension à 72° C pendant 90 secondes.

Des contrôles négatifs sont réalisés sur des échantillons d'ARN amplifiés en l'absence de transcriptase inverse. Les produits d'amplification sont séparés par électrophorèse en gel d'agarose à 1,5 % contenant du bromure d'éthidium. Les fragments d'ADN amplifiés sont visualisés sous une lampe à ultraviolet.

### 4- Amplification PCR des exons 6, 7, 9 et 12 du gène MDR3

Les exons 6, 9 et 12 sont amplifiés par PCR, en utilisant des amorces localisées dans les régions introniques flanquantes et en utilisant de l'ADN génomique comme matrice :
- un fragment de 321 pb couvrant l'exon 6 est amplifié en utilisant les amorces suivantes :
   - amorce sens 5'-CTACTCAGGATTGGGTGCTGG-3' (SEQ ID NO :1) et
   - amorce anti-sens 5'-GCTAGAACATGGCTGCCAG-3' (SEQ ID NO :2)
- un fragment de 210 pb couvrant l'exon 9 est amplifié en utilisant les amorces suivantes :
   - amorce sens 5'-CCCTCTCATTTTTCTGGTAG-3' (SEQ ID NO :3) et
   - amorce anti-sens 5'-GTTAGGAGAACTACTTACTG-3' (SEQ ID NO:4).
- un fragment de 154 pb couvrant l'exon 12 est amplifié en utilisant les amorces suivantes :
   - amorce sens 5'-CCTTACAGATCTTGAAGGGC-3' (SEQ ID NO :5) et
   - amorce anti-sens 5'-GCTTGGTTCTTCCCACTTAC-3' (SEQ ID NO :6).

La réaction de PCR est réalisée dans un volume final de 50 µl, en présence de 100 ng d'ADN génomique et de 1,5 mM de MgCl₂. L'étape d'hybridation des amorces est réalisée à 60°C (exon 7), 50°C (exon 9) et 56°C (exon 12) et les autres étapes sont identiques à celles exposées ci-dessus pour l'ADNc à l'exception du nombre de cycles (30 au lieu de 35). Les produits d'amplification sont séparés par électrophorèse en gel d'agarose à 2 % contenant du bromure d'éthidium. Les fragments d'ADN amplifiés sont visualisés sous une lampe à ultraviolet.

### 5- Analyse des mutations

Les produits d'amplification sont séquences (kit au didésoxyterminator) conformément aux instructions du fabricant (Perkin-Elmer). Les réactions de séquençage sont réalisées dans un thermocycleur 9600 Perkin-Elmer en utilisant les mêmes amorces que pour l'amplification des exons (voir 4-). Les produits d'extension sont séparés des nucléotides non-incorporés et des amorces par centrifugation sur colonne (quick spin TM, Boehringer Mannheim) puis ils sont séchés et remis en suspension dans 4 µl de formamide déionisée contenant 50 mM d'EDTA pH8. Ensuite ces produits sont chauffés pendant 2 minutes à 90°C, transférés sur de la glace et chargés immédiatement sur un gel de polyacrylamide dénaturant à 6 %. La migration se fait à 30 W pendant 12 h (séquenceur d'ADN automatisé modèle ABI 373 A).

### 6- Analyse de restriction

Les fragments d'amplification de l'exon 9 sont digérés (15 µl) pendant 6 heures à 37°C avec *Hinf I* ou *BamH I* (2 UI, Biolabs) et les fragments obtenus sont visualisés sur gels d'agarose à 2 %.

### 7- Composition de la bile

Un microscope en lumière polarisée est utilisé pour détecter la présence de cristaux de cholestérol dans des échantillons de bile fraîche.

La concentration en sels biliaires totaux est mesurée par une technique enzymatique mettant en oeuvre de la 3α-hydroxystéroïde déshydrogénase (Enzabile, Nycomed, Oslo, Norvège). Le contenu en phospholipides totaux est déterminé par une méthode enzymatique en présence de phospholipase D et de choline oxydase. Le cholestérol est déterminé par une réaction enzymatique avec de la cholestérol-oxydase ou de la cholestérol-estérase. Les indices de saturation en cholestérol des échantillons de bile hépatique sont calculés à partir des tables critiques de Carey (J. Lipid Res., 1978, 19, 945-955).

### Exemple 2 : Résultats

Les six patients précités ont été étudiés.

### 1- Morphologie et histopathologie

L'ultrasonographie (US) permet de détecter de multiples microcalculs dans les conduits biliaires intra-hépatiques chez les six patients.

Un échantillon de biopsie hépatique obtenu chez les patients 3 et 4, montre une inflammation portale modérée et une prolifération des canaux biliaires sans lésion desdits canaux. Une fibrose extensive est également observée chez le patient 4.

Pour ce qui concerne le patient 3, de nombreux microcalculs biliaires visibles à la radio ont été extraits des canaux biliaires intra-hépatiques et extra-hépatiques. L'analyse biochimique de ces calculs montre qu'ils sont constitués essentiellement de cholestérol.

Une choliangographie a été réalisée chez le patient 5 et montre une cholangite sclérosante localisée, caractérisée par des conduits biliaires intra-hépatiques gauches irréguliers présentant une alternance de zones de sténose et des zones de dilatation sacculaire.

### 2- Composition de la bile hépatique

La composition en lipides biliaires de la bile hépatique a été étudiée à partir de prélèvements obtenus par radioscopie du duodénum (patients 2 et 3) ou à partir du tube T (patients 5 et 6). Les résultats obtenus chez les patients non-traités ou sous traitement à l'AUDC sont présentés respectivement dans les tableaux II et III ci-dessous.

**Tableau II: Composition en lipides biliaires de la bile hépatique chez les patients non-traités.**

| **Paramètres mesurés** | **Valeurs normales** * | **Cas 2** | **Cas 5** | **Cas 6** |
|---|---|---|---|---|
| Classes de lipides biliaires | | | | |
| (moles %) | | | | |
| Sels biliaires (BS) | 72.6 | 72.1 | 57.3 | 94.2 |
| Phospholipides (PL) | 20.6 | 18.7 | 25.3 | 1.4 |
| Cholestérol (CH) | 7.8 | 9.1 | 17.4 | 4.5 |
| | | | | |
| Index de saturation Cholestérol | 1.1 | 1.98 | 20.9 | ND |
| (ISC) | | | | |

| Rapports lipidiques | | | | |
|---|---|---|---|---|
| CH/PL | 0.38 | 0.49 | 0.70 | 3.21 |
| PL/BS | 0.29 | 0.26 | 0.44 | 0.01 |
| CH/BS | 0,10 | 0.13 | 0.30 | 0.05 |
| Concentration en PL | 15.6 | 6.39 | 1.77 | 0.30 |
| biliaires | | | | |

| | | | | |
|---|---|---|---|---|
| * Les valeurs normales sont issues de Lamont et al., (*Progress in Liver Diseases, Boyer et al. Ed., W.B. Saunders Company,* Philadelphie, 1992, 165-191). | | | | |

**Tableau III: Composition en lipides biliaires de la bile hépatique chez les patients sous traitement à l'AUDC**

| Paramètres mesurés | Valeurs normales* | Cas 5 | Cas 6 |
|---|---|---|---|
| Classes de lipides biliaires (moles %) | | | |
| Sels biliaires (BS) | 72.6 | 47.5 | 72 |
| Phospholipides (PL) | 20.6 | 35.7 | 19.7 |
| Cholestérol (CH) | 7.8 | 16.8 | 8.2 |
| Index de saturation Cholestérol | 1.1 | 4.0 | 1.8 |
| (lSC) | | | |

| Rapports lipidiques | | | |
|---|---|---|---|
| CH/PL | 0.38 | 0.47 | 0.42 |
| PL/BS | 0.29 | 0.75 | 0.27 |
| CH/BS | 0.10 | 0.35 | 0.11 |
| Concentration en PL biliaires | 15.6 | 7.77 | 6.03 |

| | | | |
|---|---|---|---|
| * Les valeurs normales sont issues de Lamont et al. (précité). | | | |

L'analyse en microscopie des échantillons de bile montre la présence de nombreux cristaux de monohydrate de cholestérol.

● Tous les échantillons de bile, fraîchement prélevés, issus de patients non-traités à l'AUDC présentent les trois caractéristiques suivantes :
1°) un index de saturation en cholestérol (ISC) élevé,
2°) un rapport cholestérol/phospholipides (CH/PL) élevé et
3°) une faible ou une très faible concentration en phospholipides (PL), en comparaison avec les concentrations normales décrites (Lee et al., 1986, Gastroenterology, 90, 677-686 ; Lamont et al., précité).

Chez le patient 5, l'augmentation importante du rapport cholestérol/sels biliaires (CH/BS) indique que l'hypersécrétion de cholestérol est responsable de l'augmentation de la saturation en cholestérol de la bile.

Chez le patient 6, la diminution importante des rapports phospholipides/sels biliaires (PL/BS) et cholestérol/sels biliaires (CH/BS) indique que dans ce cas c'est une diminution de la sécrétion de sels biliaires qui est responsable de l'augmentation de la saturation en cholestérol de la bile.

● Après traitement à l'AUDC, on observe une diminution importante de l'index de saturation en cholestérol (patients 5 et 6), liée à une augmentation de la concentration en phospholipides de la bile. En conséquence, on observe une diminution du rapport cholestérol/sels biliaires chez le patient 5, alors que les valeurs faibles des rapports cholestérol/sels biliaires et phospholipides/sels biliaires redeviennent normaux chez le patient 6.

### 3- Analyse des mutations du gène MDR3

La séquence des fragments 2, 3, 4, 5 et 6 obtenus par RT-PCR a été analysée et les mutations observées sont les suivantes :

### ● Insertion

Chez les patients 2, et 3, on détecte l'insertion d'un seul nucléotide en position 1327 de l'exon 12, sous la forme d'une mutation hétérozygote. Cette mutation entraîne une modification de la phase ouverte de lecture au niveau du codon 443 qui entraîne l'apparition d'un codon stop prématuré et la production d'une protéine tronquée de 446 acides aminés.

### ● Mutation faux-sens

Une mutation faux-sens au niveau du codon 320 de l'exon 9 (Ser₃₂₀ (TCC) ->Phe₃₂₀ (TTC), nucléotide 959 T->C) est présente à l'état homozygote dans des individus malades indépendants issus de familles non-consanguines (patients 1 et 4). En revanche, les individus non-malades de ces deux familles sont hétérozygotes pour cette substitution. Cette mutation n'est pas détectée sur chacun des allèles de 63 témoins indépendants (126 chromosomes) ce qui démontre que cette mutation ne correspond pas à un polymorphisme du gène *MDR3.*

Une mutation faux-sens au niveau du codon 175 de l'exon 6 (Thr₁₇₅ (ACG) ->Val₁₇₅ (GCG), nucléotide 523 A->G) est présente à l'état hétérozygote chez le patient 5. Cette mutation n'est pas détectée sur chacun des allèles de 51 témoins indépendants (102 chromosomes) ce qui démontre que cette mutation ne correspond pas à un polymorphisme du gène *MDR3.*

Le séquençage des fragments amplifiés à partir de l'ADN génomique confirment l'homozygotie des patients 1 et 4 pour la mutation S320F, l'hétérozygotie des patients 2 et 3 pour la mutation 1327insA et l'hétérozygotie du patient 5 pour la mutation T175A.

La digestion des fragments amplifiés par des enzymes de restriction confirme la présence de la mutation S320F chez le patient 1 (transition T->C homozygote) et chez les membres de la même famille (transition T->C hétérozygote), ainsi que l'absence de cette mutation chez des individus normaux non-apparentés.

Aucune mutation n'a été détectée dans les exons 6, 9 et 12 du patient 6, qui présente vraisemblablement une mutation au niveau du promoteur.

## Revendications

1. Procédé de dépistage d'un syndrome hépatique associant (i) une cholestase intra-hépatique, (ii) une microlithiase biliaire cholestérolique et (iii) au moins une mutation du gène MDR3, **caractérisé en ce qu'**il comprend au moins la détection, à partir d'un échantillon d'acide nucléique extrait de cellules mononucléées du sang périphérique, d'au moins une mutation hétérozygote du gène *MDR3* et/ou d'une mutation homozygote n'abolissant pas l'expression de la protéine exprimée par ledit gène, à activité de transporteur de la phosphatidylcholine, chez des sujets adultes associant une microlithiase biliaire cholestérolique et une cholestase intra-hépatique.

2. Procédé selon la revendication 1, **caractérisé en ce que** ladite mutation est située dans l'exon 6 et/ou dans l'exon 9 et/ou dans l'exon 12.

3. Procédé selon la revendication 1 ou la revendication 2, **caractérisé en ce que** ladite mutation est sélectionnée dans le groupe constitué par une mutation dans l'exon 6 au niveau du nucléotide 523, une mutation dans l'exon 9, au niveau du nucléotide 959 et une mutation dans l'exon 12 au niveau du nucléotide 1327.

4. Procédé selon la revendication 3, **caractérisé en ce que** la mutation située au niveau du nucléotide 523 est une mutation hétérozygote faux-sens A/G (ACG/GCG), conduisant à la mutation en acide aminé T175A, la mutation située au niveau du nucléotide 959 est une mutation homozygote faux-sens C/T (TCC/TTC), conduisant à la mutation en acide aminé S320F et la mutation située au niveau du nucléotide 1327 correspond à l'insertion d'une adénine (1327insA).

5. Procédé selon l'une quelconque des revendications 1 à 4, **caractérisé en ce qu'**il comprend :
- une première étape d'amplification d'un fragment d'acide nucléique, dans lequel l'une au moins desdites mutations est susceptible d'être observée, à partir des acides nucléiques extraits de cellules mononucléées du sang périphérique, à l'aide d'au moins une amorce sélectionnée dans le groupe constitué par les amorces présentant les séquences SEQ ID NO:1 à SEQ ID NO: 13 et
- une deuxième étape de détection de la présence d'au moins l'une desdites mutations, à partir du ou desdits fragments d'amplification obtenus.

6. Procédé selon la revendication 5, **caractérisé en ce que** la première étape d'amplification consiste en une amplification PCR directe d'au moins l'un des exons 6, 9 et 12 de l'ADN génomique avec les amorces suivantes localisées dans les régions introniques flanquantes :
- amorces SEQ ID NO: 1 et SEQ ID NO:2, qui permettent d'amplifier un fragment de 321 pb couvrant l'exon 6,
- amorces SEQ ID NO:3 et SEQ ID NO:4, qui permettent d'amplifier un fragment de 210 pb couvrant l'exon 9, et
- amorces SEQ ID NO:5 et SEQ ID NO:6, qui permettent d'amplifier un fragment de 154 pb couvrant l'exon 12.

7. Procédé selon la revendication 5, **caractérisé en ce que** la première étape d'amplification consiste en une amplification RT-PCR des ARNm par deux PCR imbriquées, à l'aide de deux paires d'amorces, sélectionnées dans le groupe constitué par les paires suivantes :
- SEQ ID NO:7 et SEQ ID NO:8
- SEQ ID NO:9 et SEQ ID NO:10
- SEQ ID NO:11 et SEQ ID NO:10
- SEQ ID NO:12 et SEQ ID NO:10 et
- SEQ ID NO:13 et SEQ ID NO:10.

8. Procédé selon l'une quelconque des revendications 5 à 7, **caractérisé en ce que** la deuxième étape de détection de la présence d'au moins l'une desdites mutations à partir du fragment amplifié, est réalisée selon le cas, par l'une des méthodes suivantes : séquençage, restriction enzymatique ou techniques de type PCR/PCR/LDR.

9. Procédé selon la revendication 6, **caractérisé en ce que** l'étape de détection est effectuée par digestion du fragment d'amplification de 210 pb avec l'enzyme *Hinf.I*

10. Procédé selon la revendication 6, **caractérisé en ce que** l'étape de détection est effectuée par digestion du fragment d'amplification de 210 pb avec l'enzyme *BamHI.*

11. Utilisation du procédé selon l'une quelconque des revendications 1 à 10, pour l'évaluation du risque d'apparition d'un syndrome associant une cholestase intra-hépatique et une micro lithiase biliaire cholestérolique dans les familles à risque de lithiase cholestérolique et/ou ayant des anomalies biologiques hépatiques inexpliquées.

12. Utilisation des amorces sélectionnées dans le groupe constitué par les séquences SEQ ID NO:1 à SEQ ID NO:13 pour le dépistage d'un syndrome associant une microlithiase biliaire cholestérolique, une cholestase intra-hépatique et au moins une mutation du gène *MDR3.*

13. Kit pour le dépistage d'un syndrome associant une microlithiase biliaire cholestérolique, une cholestase intra-hépatique et au moins une mutation du gène *MDR3,* **caractérisé en ce qu'**il est constitué par les trois paires d'amorces suivantes :
- amorce de séquence SEQ ID NO : 1 et amorce de séquence SEQ ID NO : 2 ;
- amorce de séquence SEQ ID NO : 3 et amorce de séquence SEQ ID NO : 4 ; et
- amorce de séquence SEQ ID NO : 5 et amorce de séquence SEQ ID NO : 6.

14. Kit pour l'évaluation du risque d'apparition d'un syndrome associant une microlithiase biliaire cholestérolique, une cholestase intra-hépatique et au moins une mutation du gène *MDR3* dans une population à risque, **caractérisé en ce qu'**il comprend outre les trois paires d'amorces suivantes :
- amorce de séquence SEQ ID NO : 1 et amorce de séquence SEQ ID NO : 2 ;
- amorce de séquence SEQ ID NO : 3 et amorce de séquence SEQ ID NO : 4 ; et
- amorce de séquence SEQ ID NO : 5 et amorce de séquence SEQ ID NO : 6 ; des réactifs pour doser le cholestérol biliaire et les phospholipides biliaires.

15. Utilisation de l'acide ursodésoxycholique pour la préparation d'un médicament destiné à traiter un syndrome associant une microlithiase biliaire cholestérolique, une cholestase intra-hépatique et au moins une mutation du gène *MDR3.*

## Claims

1. Process for screening for a hepatic syndrome which combines (i) an intrahepatic cholestasis, (ii) a cholesterol biliary microlithiasis and (iii) at least one mutation of the MDR3 gene, **characterised in that** it comprises at least detecting, from a sample of nucleic acid extracted from mononucleated cells from the peripheral blood, at least one heterozygotic mutation of the *MDR3* gene and/or a homozygotic mutation which does not stop the expression of the protein expressed by said gene, with a phosphatidylcholine-transporting activity, in adult subjects who have a combination of a cholesterol biliary microlithiasis and an intrahepatic cholestasis.

2. Process according to claim 1, **characterised in that** said mutation is located in exon 6 and/or in exon 9 and/or in exon 12.

3. Process according to claim 1 or claim 2, **characterised in that** said mutation is selected from the group consisting of a mutation in exon 6 at nucleotide 523, a mutation in exon 9 at nucleotide 959 and-a mutation in exon 12 at nucleotide 1327.

4. Process according to claim 3, **characterised in that** the mutation at nucleotide 523 is an anti-sense A/G (ACG/GCG) heterozygotic mutation, leading to the amino acid mutation T175A, the mutation at nucleotide 959 is an anti-sense C/T (TCC/TTC) homozygotic mutation leading to the amino acid mutation S320F and the mutation at nucleotide 1327 corresponds to the insertion of an adenine (1327insA).

5. Process according to any one of claims 1 to 4, **characterised in that** it comprises:
- a first step of amplifying a nucleic acid fragment wherein at least one of said mutations is likely to be observed, from nucleic acids extracted from peripheral blood mononuclear cells, using at least one primer selected from the group consisting of the primers having sequences SEQ ID NO:1 to SEQ ID NO:13 and
- a second step of detecting the presence of at least one of said mutations, from the amplification fragment or fragments obtained.

6. Process according to claim 5, **characterised in that** the first amplification step consists of a direct PCR amplification of at least one of exons 6, 9 and 12 of the genomic DNA with the following primers located in the flanking intron regions:
- primers SEQ ID NO:1 and SEQ ID NO:2, which can be used to amplify a 321 bp fragment covering exon 6,
- primers SEQ ID NO:3 and SEQ ID NO:4, which can be used to amplify a 210 bp fragment covering exon 9, and
- primers SEQ ID NO:5 and SEQ ID NO:6, which can be used to amplify a 154 bp fragment covering exon 12.

7. Process according to claim 5, **characterised in that** the first amplification step comprises RT-PCR amplification of the mRNAs by two nested PCRs, using two pairs of primers selected from the group consisting of the following pairs:
SEQ ID NO:7 and SEQ ID NO: 8
SEQ ID NO:9 and SEQ ID NO:10
SEQ ID NO: 11 and SEQ ID NO:10
SEQ ID NO: 12 and SEQ ID NO:10 and
SEQ ID NO:13 and SEQ ID NO:10.

8. Process according to any one of claims 5 to 7, **characterised in that** the second step of detecting the presence of at least one of said mutations from the amplified fragment is carried out by one of the following methods, depending on the circumstances: sequencing, enzymatic restriction or techniques of the PCR/PCR/LDR type.

9. Process according to claim 6, **characterised in that** the detection step is carried out by digesting the 210 bp amplification fragment with the enzyme *HinfI.*

10. Process according to claim 6, **characterised in that** the detection step is carried out by digesting the 210 bp amplification fragment with the enzyme *BamHI.*

11. Use of the process according to any one of claims 1 to 10, for evaluating the risk of the occurrence of a syndrome combining an intrahepatic cholestasis and a cholesterol biliary microlithiasis in families at risk of cholesterol lithiasis and/or having unexplained biological hepatic anomalies.

12. Use of primers selected from among the group consisting of the sequences SEQ ID NO: 1 to SEQ ID NO:13 for screening for a syndrome combining a cholesterol biliary microlithiasis, an intrahepatic cholestasis and at least one mutation of the gene *MDR3.*

13. Kit for screening for a syndrome combining cholesterol biliary microlithiasis, intrahepatic cholestasis and at least one mutation of the gene *MDR3,* **characterised in that** it consists of the following three pairs of primers:
- sequence primer SEQ ID NO:1 and sequence primer SEQ ID NO:2;
- sequence primer SEQ ID NO:3 and sequence primer SEQ ID NO:4; and
- sequence primer SEQ ID NO:5 and sequence primer SEQ ID NO:6.

14. Kit for evaluating the risk of the occurrence of a syndrome combining a cholesterol biliary microlithiasis, an intrahepatic cholestasis and at least one mutation of the gene *MDR3* in a population at risk, **characterised in that** it comprises in addition to the following three pairs of primers:
- sequence primer SEQ ID NO:1 and sequence primer SEQ ID NO:2;
- sequence primer SEQ ID NO:3 and sequence primer SEQ ID NO:4; and
- sequence primer SEQ ID NO:5 and sequence primer SEQ ID NO:6; reagents for measuring the bile cholesterol and the bile phospholipids.

15. Use of ursodeoxycholic acid for preparing a medicament intended for treating a syndrome combining a cholesterol biliary microlithiasis, an intrahepatic cholestasis and at least one mutation of the gene *MDR3.*

## Patentansprüche

1. Verfahren zur Feststellung eines hepatischen Syndroms, einhergehend mit:
(i) einer intrahepatischen Cholestase,
(ii) einer biliären Mikrocholelithiasis und
(iii) wenigstens einer Mutation des Gens *MDR3,* **dadurch gekennzeichnet, dass** es wenigstens die Erkennung ausgehend von einer Nukleinsäureprobe, extrahiert aus einkernigen Zellen peripheren Bluts, von wenigstens einer heterozygoten Mutation des Gens *MDR3* und/oder von einer homozygoten Mutation, welche die Expression des exprimierten Proteins für die Aktivität des Phosphatidylcholin-Transporters bei erwachsenen Probanden mit einer biliären Mikrocholelithiasis und einer intrahepatischen Cholestase durch das besagte Gen nicht verhindert, umfasst.

2. Verfahren nach Anspruch 1, **dadurch gekennzeichnet, dass** die besagte Mutation in dem Exon 6 und/oder in dem Exon 9 und/oder in dem Exon 12 vorliegt.

3. Verfahren nach Anspruch 1 oder Anspruch 2, **dadurch gekennzeichnet, dass** die besagte Mutation ausgewählt wird aus der Gruppe bestehend aus einer Mutation in dem Exon 6 auf Höhe des Nukleotids 523, einer Mutation in dem Exon 9 auf Höhe des Nukleotids 959 sowie einer Mutation in dem Exon 12 auf Höhe des Nukleotids 1327.

4. Verfahren nach Anspruch 3, **dadurch gekennzeichnet, dass** die Mutation, welche auf Höhe des Nukleotids 523 vorliegt, eine heterozygote antisense-Mutation von A/G (ACG/GCG) ist, welche zu einer Aminosäuremutation T175A führt, die Mutation, weiche auf Höhe des Nukleotids 959 vorliegt, eine homozygote antisense-Mutation von C/T (TCC/TTC) ist, die zu der Aminosäuremutation S320F führt, und die Mutation, welche auf Höhe des Nukleotids 1327 vorliegt, der Inserierung eines Adenins entspricht (1327insA)

5. Verfahren nach irgendeinem der Ansprüche 1 bis 4, **dadurch gekennzeichnet, dass** es umfasst:
- eine erste Amplifikationsstufe eines Nukleinsäurefragments, in welchem wenigstens eine der besagten Mutationen beobachtet werden kann, ausgehend von Nukleinsäuren, extrahiert aus einkernigen Zellen des peripheren Bluts unter Zuhilfenahme wenigstens eines Primers, ausgewählt aus der Gruppe bestehend aus Primern, welche die Sequenzen SEQ ID NO: 1 bis SEQ ID NO: 13 darstellen, und
- eine zweite Erfassungsstufe für das Vorliegen wenigstens einer der besagten Mutationen, ausgehend von den dort erhaltenen oder besagten Amplifikationsfragmenten.

6. Verfahren nach Anspruch 5, **dadurch gekennzeichnet, dass** die erste Amplifikationsstufe aus einer direkten PCR-Amplifikation besteht mit wenigstens einem der Exons 6, 9 und 12 der genomischen DNS mit folgenden Primern, welche in den flankierenden Intronbereichen angeordnet sind:
- Primer SEQ ID NO: 1 und SEQ lD NO: 2, welche die Amplikation eines Fragments mit 321 bp, welches das Exon 6 abdeckt, erlauben,
- Primer SEQ ID NO: 3 und SEQ ID NO: 4, welche die Amplifikation eines Fragments mit 210 bp, welches das Exon 9 abdeckt, erlauben, und
- Primer SEQ ID NO: 5 und SEQ ID NO: 6, welche die Amplifikation eines Fragments mit 154 bp, welches das Exon 12 abdeckt, erlauben.

7. Verfahren nach Anspruch 5, **dadurch gekennzeichnet, dass** die erste Amplifikationsstufe aus einer RT-PCR-Amplifikation von mRNA durch zwei sich überschneidende PCR besteht unter Zuhilfenahme von zwei Primerpaaren, ausgewählt aus der Gruppe bestehend aus den folgenden Paaren:
- SEQ ID NO: 7 und SEQ ID NO: 8
- SEQ ID NO: 9 und SEQ ID NO: 10
- SEQ ID NO: 11 und SEQ ID NO; 10
- SEQ ID NO: 12 und SEQ ID NO: 10, und
- SEQ ID NO: 13 und SEQ 10 NO: 10.

8. Verfahren nach irgendeinem der Ansprüche 5 bis 7, **dadurch gekennzeichnet, dass** die zweite Erfassungsstufe für das Vorliegen wenigstens eines der besagten Mutationen ausgehend von dem amplifizierten Fragment von Fall zu Fall durch eines der folgenden Verfahren realisiert ist: Sequenzierung, enzymatische Restriktion oder Techniken vom Typ PCR/PCR/LDR.

9. Verfahren nach Anspruch 6, **dadurch gekennzeichnet, dass** die Erfassungsstufe bewirkt wird durch Verdau des Amplifikationsfragments mit 210 bp mit dem Enzym *Hinfl.*

10. Verfahren nach Anspruch 6, **dadurch gekennzeichnet, dass** die Erfassungsstufe bewirkt wird durch Verdau des Amplifikationsfragments mit 210 bp mit dem Enzym *BamHI.*

11. Verwendung eines Verfahrens gemäß irgendeinem der Ansprüche 1 bis 10 zur Abschätzung des Risikos für das Auftreten eines Syndroms, welches einhergeht mit einer intrahepatischen Cholestase und einer biliären Mikrocholelithiasis in Familien mit einem Cholelithiasisrisiko und/oder in denen ungeklärte biologische hepatische Anomalien auftreten.

12. Verwendung von Primern, ausgewählt aus der Gruppe bestehend aus den Sequenzen SEQ ID NO: 1 bis SEQ ID NO: 13 zur Feststellung eines Syndroms, einhergehend mit einer biliären Mikrocholelithiasis, einer intrahepatischen Cholestase und wenigstens einer Mutation des Gens *MDR3.*

13. Kit zum Feststellen eines Syndroms, einhergehend mit einer biliären Mikrocholelithiasis, einer intrahepatischen Cholestase und wenigstens einer Mutation des Gens *MDR3,* **dadurch gekennzeichnet, dass** er aufgebaut ist aus den drei folgenden Primerpaaren:
- Primer der Sequenz SEQ ID NO: 1 und Primer der Sequenz SEQ ID NO: 2;
- Primer der Sequenz SEQ ID NO: 3 und Primer der Sequenz SEQ ID NO: 4; und
- Primer der Sequenz SEQ ID NO: 5 und Primer der Sequenz SEQ ID NO: 6.

14. Kit zur Abschätzung eines Risikos für das Auftreten eines Syndroms, einhergehend mit einer biliären Mikrocholelithiasis, einer intrahepatischen Cholestase und wenigstens einer Mutation des Gens *MDR3* in einer Risikopopulation, **dadurch gekennzeichnet, dass** er die folgenden drei weiteren Primerpaare enthält:
- Primer der Sequenz SEQ ID NO: 1 und Primer der Sequenz SEQ ID NO: 2;
- Primer der Sequenz SEQ ID NO: 3 und Primer der Sequenz SEQ ID NO: 4; und
- Primer der Sequenz SEQ ID NO: 5 und Primer der Sequenz SEQ ID NO: 6;
Reagenzien zum Dosieren von biliärem Cholesterol und der biliären Phospholipide.

15. Verwendung der Ursodesoxycholsäure für die Herstellung eines Medikaments zur Behandlung eines Syndroms, welches mit einer biliären Mikrocholelithiasis, einer intrahepatischen Cholestase und wenigstens einer Mutation des Gens *MDR3* einhergeht.


### LISTE DE SEQUENCES

<110> ASSISTANCE PUBLIQUE-HOPITAUX DE PARIS
   POUPON Raoul
   HERMELIN Brigitte
   ROSMORDUC Olivier
<120> DEPISTAGE D'UN NOUVEAU SYNDROME HEPATIQUE ET SES APPLICATIONS.
<130> BLOcp1020CAS13
<140>
   <141>
<160> 14
<170> PatentIn Ver. 2.1
<210> 1
   <211> 21
   <212> ADN
   <213> Séquence artificielle
<220>
   <223> Description de la séquence artificielle:AMORCE
<400> 1
   ctactcagga ttgggtgctg g 21
<210> 2
   <211> 19
   <212> ADN
   <213> Séquence artificielle
<220>
   <223> Description de la séquence artificielle:AMORCE
<400> 2
   gctagaacat ggctgccag 19
<210> 3
   <211> 20
   <212> ADN
   <213> Séquence artificielle
<220>
   <223> Description de la séquence artificielle:AMORCE
<400> 3
   ccctctcatt tttctggtag 20
<210> 4
   <211> 20
   <212> ADN
   <213> Séquence artificielle
<220>
   <223> Description de la séquence artificielle:AMORCE
<400> 4
   gttaggagaa ctacttactg 20
<210> 5
   <211> 20
   <212> ADN
   <213> Séquence artificielle
<220>
   <223> Description de la séquence artificielle:AMORCE
<400> 5
   ccttacagat cttgaagggc 20
<210> 6
   <211> 20
   <212> ADN
   <213> Séquence artificielle
<220>
   <223> Description de la séquence artificielle:AMORCE
<400> 6
   gcttggttct tcccacttac 20
<210> 7
   <211> 23
   <212> ADN
   <213> Séquence artificielle
<220>
   <223> Description de la séquence artificielle:AMORCE
<400> 7
   cctgccagac acgcgcgagg ttc 23
<210> 8
   <211> 25
   <212> ADN
   <213> Séquence artificielle
<220>
   <223> Description de la séquence artificielle:AMORCE
<400> 8
   cttcaagtcc atcggtttcc acatc 25
<210> 9
   <211> 19
   <212> ADN
   <213> Séquence artificielle
<220>
   <223> Description de la séquence artificielle:AMORCE
<400> 9
   ccttgtcgct gctaaatcc 19
<210> 10
   <211> 21
   <212> ADN
   <213> Séquence artificielle
<220>
   <223> Description de la séquence artificielle:AMORCE
<400> 10
   ggctcttctg acacatttgt g 21
<210> 11
   <211> 20
   <212> ADN
   <213> Séquence artificielle
<220>
   <223> Description de la séquence artificielle:AMORCE
<400> 11
   ggaattggtg acaaggttgg 20
<210> 12
   <211> 25
   <212> ADN
   <213> Séquence artificielle
<220>
   <223> Description de la séquence artificielle:AMORCE
<400> 12
   gctatttcag caaacatttc catgg 25
<210> 13
   <211> 22
   <212> ADN
   <213> Séquence artificielle
<220>
   <223> Description de la séquence artificielle:AMORCE
<400> 13
   gctaacgtca agatcttgaa gg 22
<210> 14
   <211> 18
   <212> ADN
   <213> Séquence artificielle
<220>
   <223> Description de la séquence artificielle: amorce
<400> 14
   gaggccaacg cctatgag 18